# EUROPEAN PATENT SPECIFICATION

(11) **EP 1 986 011 B1**
(45) Date of publication and mention of the grant of the patent: **11.02.2015**
(21) Application number: 08006581.6
(22) Date of filing: 20.04.2000
(51) Int. Cl.: G01N 33/68

(54) **Peptide epitopes recognized by disease promoting CD4+ T lymphocytes**
Von krankheits-fördernden CD4+ T- Lymphozyten erkannte Peptidepitope
Epitopes peptidiques identifiés par des lymphocytes T CD4+ stimulés par la maladie

(30) Priority: 21.04.1999 US 295868; 21.04.1999 US 130355 P
(43) Date of publication of application: 29.10.2008
(62) Divisional of application: 00926280.9
(73) Proprietor: Eisai Inc., Woodcliff Lake, NJ 07677 (US); King's College London, London WC2R 2LS (GB)
(72) Inventor: Peakman, Mark, London SE24 9LG (GB); Chicz, Roman M., Belmont Massachusetts 02478 (US)
(74) Representative: Tombling, Adrian George

(56) References cited:
- US-A- 5 827 516
- HONEYMAN, M.C. ET AL.: "T-cell epitopes in type 1 dibetes autoantigen tyrosine phophatase IA-2: potential for mimicry with rotavirus and other environmental agents.", MOL. MED., vol. 4, no. 4, 1998, pages 231-239, XP000938317,
- LOHMANN, T. ET AL.: "T-cell reactivity to DR*0401- and DQ*0302-binding peptides of the putative autoantigen IA-2 in type 1 diabetes.", EXP. CLIN. ENDOCRINOL. DIABETES, vol. 107, no. 3, 1999, pages 166-171, XP000938310,
- STEVENS, E.J. AND PEAKMAN, M.: "Enhanced T cell proliferation and increased responder frequency following delivery of antigen to the antigen-presenting cell; B cell dependency and use in detection of autoreactive cells", JOURNAL OF IMMUNOLOGICAL METHODS, vol. 215, 1998, pages 59-70, XP002147848,
- PEAKMAN, MARK ET AL: "Naturally processed and presented epitopes of the islet cell autoantigen IA-2 eluted from HLA-DR4", J. CLIN. INVEST. (1999), 104(10), 1449-1457, 1999, XP002147849,
- NIJMAN H W ET AL: "CHARACTERIZATION OF CYTOTOXIC T LYMPHOCYTE EPITOPES OF A SELF-PROTEIN, P53, AND A NON-SELF-PROTEIN, INFLUENZA MATRIX: RELATIONSHIP BETWEEN MAJOR HISTOCOMPATIBILITY COMPLEX PEPTIDE BINDING AFFINITY AND IMMUNE RESPONSIVENESS TO PEPTIDES", JOURNAL OF IMMUNOTHERAPY,US,RAVEN PRESS, NEW YORK, vol. 14, no. 2, 1993, pages 121-126, XP002015226, ISSN: 1053-8550
- HONEYMAN, M.C. ET AL.: "Strategies for identifying and predicting islet autoantigen T-cell epitopes in insulin-dependent diabetes mellitus", ANN MED, vol. 29, no. 5, 1997, pages 401-404, XP000938321,

## Description

### Background of the Invention

The invention is in the field of diseases with an immunological aetiology, particularly diseases involving CD4+ T lymphocytes.

After internalization and proteolytic processing of intact protein antigens by antigen presenting cells (APCs) class II Major Histocompatibility Complex (MHC) molecules on the APCs bind short antigenic peptides (epitopes) derived from the antigens, presenting the bound peptides to CD4+ T lymphocytes [Germain, R.N. (1994), Cell 76:287-299]. Class II MHC genes and the molecules they encode are highly variable between individuals, and differences between the class II MHC molecules have profound effects on which peptides are selected for presentation as T cell epitopes. The different forms (alleles) of class II MHC molecules expressed by an individual have a major effect on the individual's susceptibility to a range of CD4+ T cell-mediated diseases, most notably autoimmune disease such as insulin dependent diabetes mellitus (IDDM) [Davies et al. (1994), Nature 371:130-136]. It is important that the antigenic epitopes of antigens recognized by the CD4+ T cells mediating these diseases be defined in order to develop effective therapeutic and/or prophylactic products and protocols.

Honeyman et al., (Molecular Medicine, 4 231-239, 1998) and Lohmann et al., (Exp. Clin. Endocrinol. Diabetes, 107, 166-171, 1999) disclose a number of IA-2 peptides that elicit weaker immune responses in patients compared to the peptides of the present invention.

### Summary of the Invention

The invention features peptide epitopes that activate CD4+ T lymphocyte responses involved in the initiation, promotion, or exacerbation of certain diseases, especially those in which susceptibility is determined by expression of defined class II MHC molecules. The invention is based on the discovery that artificially binding a polypeptide molecule to the cell membrane of an APC facilitates transport of the molecule to the antigen processing organelles of the APC. The invention is directed to peptides derived from the diabetes autoantigen, IA-2.

The invention is an isolated peptide that binds to HLA-DR4, consisting of the - amino acid sequence:
(1) YLKNVQTQETRTL (SEQ ID NO: 10);
(2) YLKNVQTQETRTLTQ (SEQ ID NO: 13); or
(3) FYLKNVQTQETRTLTQFHF (SEQ ID NO: 16).

The invention also includes an isolated peptide that binds to HLA-DR4, consisting of the amino acid sequence:
(1) AYQAEPNTCATAQ (SEQ ID NO: 17);
(2) LAKEWQALCAYQAEPNT (SEQ ID NO: 19);
(3) AYQAEPNTCATAQGEGNIK (SEQ ID NO: 20);
(4) WQALCAYQAEPNTCATAQ (SEQ ID NO: 21); or
(5) LAKEWQALCAYQAEPNTCATAQGE (SEQ ID NO: 22).

The invention also includes an isolated peptide consisting of the amino acid sequence KLKVESSPSRSDYINAS (SEQ ID NO: 40).

The invention also features a method of protecting a subject from IDDM or the pathogenic symptoms of IDDM. It is understood that protecting includes alleviating (or decreasing) as well as eliminating the pathogenic symptoms in a subject. The method includes administering any of the above peptides of the invention to the subject by any of the routes disclosed herein.

Another embodiment of the invention is a method of diagnosing IDDM comprising: (a) providing CD4 lymphocytes from an individual suspected of having or being susceptible to IDDM; (b) providing a population of APCs which bear on their surface a class II MHC molecule of an allele identical to one expressed by the individual, the population of APCs having been contacted with an IA-2 peptide and the class II MHC molecule of the APCs being bound to the IA-2 peptide; (c) contacting the population of APCs of (b) with the CD4 lymphocytes of (a); and (d) determining whether the CD4 lymphocytes recognize the class II MHC-bound peptide, as an indication that the individual has or is susceptible to IDDM. The IA-2 peptide used in the method has the amino acid sequence selected from the group consisting of: YLKNVQTQETRTL (SEQ ID NO: 10); YLKNVQTQETRTLTQ (SEQ ID NO: 13); FYLKNVQTQETRTLTQFHF (SEQ ID NO: 16); AYQAEPNTCATAQ (SEQ ID NO: 17); LAKEWQALCAYQAEPNT (SEQ ID NO: 19); AYQAEPNTCATAQGEGNIK (SEQ ID NO: 20); WQALCAYQAEPNTCATAQ (SEQ ID NO: 21); LAKEWQALCAYQAEPNTCATAQGE (SEQ ID NO:22); and KLKVESSPSRSDYINAS (SEQ ID NO:40).

An "isolated" peptide of the invention is a peptide which either has no naturally-occurring counterpart (e.g., such as an APL), or has been separated or purified from components which naturally accompany it, e.g., in tissues such as pancreas, liver, spleen, ovary, testis, muscle, joint tissue, neural tissue, gastrointestinal tissue, or body fluids such as blood, serum, or urine. Typically, the peptide is considered "isolated" when it is at least 70%, by dry weight, free from the proteins and naturally-occurring organic molecules with which it is naturally associated. Preferably, a preparation of a peptide of the invention is at least 80%, more preferably at least 90%, and most preferably at least 99%, by dry weight, the peptide of the invention. Thus, for example, a preparation of peptide x is at least 80%, more preferably at least 90%, and most preferably at least 99%, by dry weight, peptide x. Since a peptide that is chemically synthesized is, by its nature, separated from the components that naturally accompany it, the synthetic peptide is "isolated."

An isolated peptide of the invention can be obtained, for example, by extraction from a natural source (e.g., from human tissues or bodily fluids); by expression of a recombinant nucleic acid encoding the peptide; or by chemical synthesis. A peptide that is produced in a cellular system different from the source from which it naturally originates is "isolated," because it will be separated from components which naturally accompany it. The extent of isolation or purity can be measured by any appropriate method, e.g., column chromatography, polyacrylamide gel electrophoresis, or HPLC analysis.

As used herein, "protection from a mammalian disease" means prevention of onset of a mammalian disease or lessening the severity of a disease existing in a mammal. "Prevention" can include a delay of onset, as well as a partial or complete block in progress of the disease.

As used herein, "a naturally-processed, diabetes-associated peptide fragment" is a peptide fragment produced by proteolytic degradation of a protein (e.g., insulin, proinsulin, preproinsulin, IA-2, IA-2β, or GAD65) in an antigen presenting cell of a mammal. Recognition of such a peptide by CD4 T cells of a mammal (e.g., a human patient) is indicative of the existence, or future onset, of diabetes in the mammal.

Unless otherwise defined, all technical and scientific terms used herein have the same meaning as commonly understood by one of ordinary skill in the art to which this invention pertains. In case of conflict, the present document, including definitions, will control. Preferred methods and materials are described below, although methods and materials similar or equivalent to those described herein can be used in the practice or testing of the present invention. Unless otherwise indicated, these materials and methods are illustrative only and are not intended to be limiting. All publications, patent applications, patents and other references mentioned herein are illustrative only and not intended to be limiting.

Other features and advantages of the invention, will be apparent from the following description, from the drawings and from the claims.

### Brief Description of the Drawings

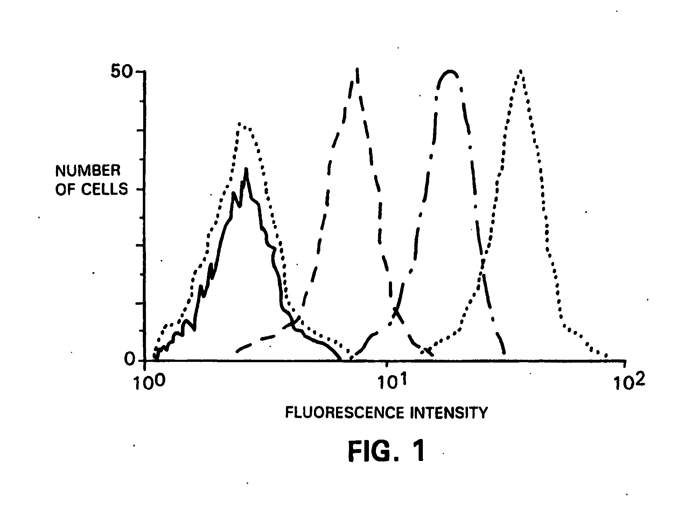

Fig. 1 is a histogram showing the fluorescence-activated flow cytometric profiles of Priess cells that were stained with tetanus toxoid specific rabbit antibody and FITC-conjugated goat antibody-specific for rabbit immunoglobulin at various times after being subjected to the Antigen Delivery System (ADS) using tetanus toxoid as the polypeptide antigen.

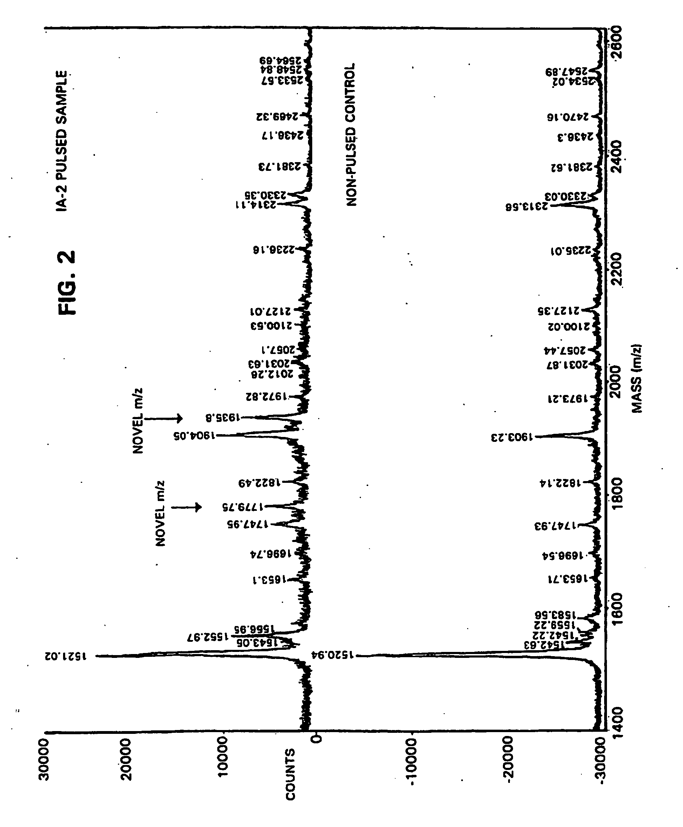

Fig. 2 is a diagram showing 2 appropriately aligned MALDI-TOF spectra derived from 2 mixtures of peptides obtained by IMF procedures in which separate aliquots of Priess cells were treated with either all the steps of the Immunological Mass Fingerprinting (IMF) procedure, including exposure to biotinylated IA-2ic, or all the steps of the IMF procedure, except exposure to biotinylated IA-2ic.

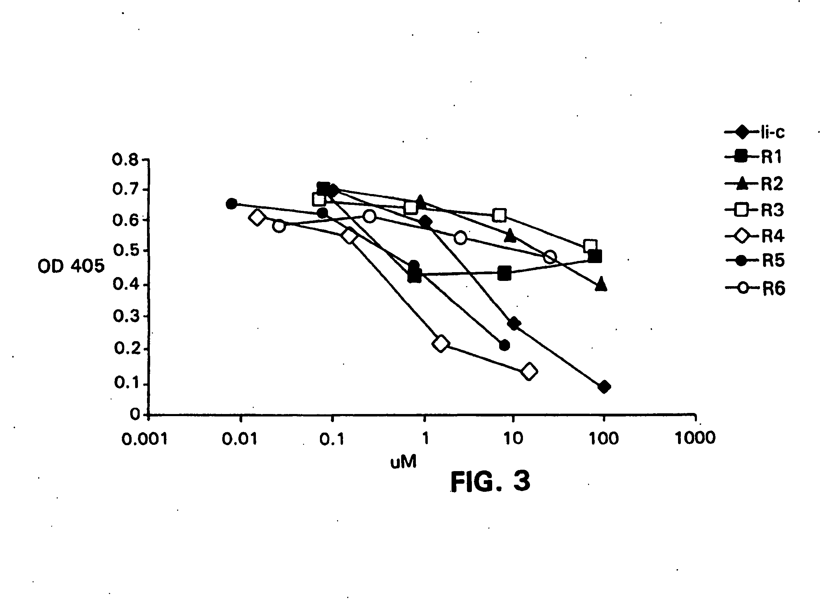

Fig. 3 is a series of line graphs showing the relative ability of 6 peptides (with amino acid sequences based on the 6 core regions of IA-2 identified by IMF) to inhibit binding of an invariant chain (Ii) peptide to isolated HLA DR4 molecules.

### Detailed Description

The present invention is based on the novel discovery that, by artificially binding a polypeptide of interest (PPI) to the cell membrane of an APC, the APC will transport the PPI to one of the antigen processing organelles within the cell, e.g., endosomes, lysozomes and structures designated "MHC class II compartments" (MIIC) that have lysosomal characteristics and are enriched for class II MHC molecules but are substantially devoid of class I MHC molecules [Peter et al. (1991), Nature 349:669-676]. The PPI is then degraded by proteolytic enzymes into peptide fragments. If any of these peptide fragments has the ability to bind to one of the class II MHC molecules expressed by the individual from which the APC was derived, it will do so in the antigen processing organelle. The resulting peptide-class II MHC molecular complex is then transported to the cell membrane, where it becomes available for interaction with CD4+ T cells bearing antigen specific receptors that specifically recognize that particular peptide-class II MHC complex. By eluting peptides from class II MHC molecules isolated from these APC, a set of naturally processed peptides derived from the PPI, as well as from other polypeptides of intracellular or extracellular origin, is obtained. The peptides, which are specific to the particular class II MHC molecules expressed by the APC, are then chemically separated and their amino acid sequences determined. By comparison of the peptide amino acid sequences to the sequence of the PPI, it is possible to identify those which are derived from the PPI. Thus, the discovery provides a method of identifying peptide fragments that are naturally processed by APC and have intrinsic binding affinity for the relevant class II MHC molecule. The method can be invaluable for identifying peptides derived from a polypeptide suspected of being an antigen that activates CD4+ T cells involved in either (a) the pathogenesis (pathology) of a disease, especially one in which susceptibility or protection is associated with expression of a particular type of class II MHC molecule, or (b) prevention or reduction of the symptoms of a disease, especially one in which protection or a reduction in severity is associated with expression of a particular type of class II MHC molecule. The method is designated "Immunological Mass Fingerprinting."

The described method ensures that the peptides identified are those that both (i) are naturally processed in vivo by the APC, and (ii) become associated, in the APC, with the relevant class II MHC molecules.

Furthermore, the present method controls for class II MHC type, an important aspect essential to link any given peptide to a particular CD4+ T cell-mediated disease in a given individual but especially important in disorders in which class II MHC type determines disease susceptibility or resistance.

Any naturally processed peptide with a sequence that corresponds to a fragment of the PPI, and which binds to a class II MHC molecule associated with the disease of interest, could be a peptide that activates CD4+ T cells that either initiate, promote, or exacerbate the disease or mediate immunity to it. To obtain confirmatory evidence of this possibility, test CD4+ T cells from subjects expressing the relevant class II MHC molecules can be assayed for responsiveness to a peptide. Control CD4+ T cells can be from subjects also expressing the class II MHC molecule but without symptoms of the disease. A significant response of the test CD4+ T cells and no response of the control CD4+ T cells would indicate that the relevant peptide is involved in the disease process (pathology of the disease) or immunity to the disease. The cellular response phase of the method is designated "Epitope Verification" ("EV").

By applying the methods to the intracellular portion of the diabetes autoantigen IA-2, IA-2-derived peptides were identified as epitopes that could be involved in the pathogenesis of diabetes in human IDDM patients expressing the DR4 class II MHC allele. Based on their amino acid sequences, these peptides fall into 6 nested groups. A consensus peptide corresponding to the core regions of each nested group was synthesized and tested for its ability to activate CD4+ T cells from either DR4-expressing IDDM patients or DR4-expressing subjects without disease symptoms. Significant responses to at least 1 out of the 6 peptides were detected in peripheral blood lymphocytes of 9/13 DR4-expressing IDDM patients. T cells from none of the control subjects responded to any of the peptides and T cells from 1 of 8 DR4 non-expressing IDDM patients responded to any peptide. These findings suggest that the 6 peptides to which the DR4-expressing IDDM patients responded represent core epitopes capable of binding to DR4 molecules and activating diabetogenic CD4+ T cells in IDDM patients. The ability of all 6 peptides to bind to isolated DR4 molecules was confirmed in an *in vitro* binding assay.

The methods can be applied to identifying peptides involved in the pathogenesis of or protection from any of a wide range of diseases, especially those in which relative susceptibility or resistance has been associated with expression of a particular class II MHC allele, provided that the amino acid sequence (or partial amino acid sequence) of a suspect polypeptide antigen is available. Candidate diseases include, without limitation, infectious diseases (e.g., diseases caused by *Chlamydia trachomatis, Helicobacter pylori, Neisseria meningitidis, Mycobacterium leprae, M. tuberculosis*, Measles virus, hepatitis C virus, human immunodeficiency virus, and *Plasmodium falciparium*), cancer (e.g. melanoma, ovarian cancer, breast cancer, colon cancer and B cell lymphomas) [Topalian, S.L. (1994), Curr. Opinion in Immunol. 6: 741-745; Topalian et al. (1996), J. Exp. Med. 183: 1965-1971], and autoimmune diseases (e.g., IDDM, rheumatoid arthritis, multiple sclerosis, myasthenia gravis, and systemic lupus erythmatosus).

The invention includes specific peptides derived from IA-2 using the above method, as described in Example 1.

### 1. Methods of Identifying CD4+ T Cell Activating Peptide Epitopes Derived From Polypeptide Antigens

The methods have two distinct phases. The first is termed "Immunological Mass Fingerprinting" (IMF) and the second the "Epitope Verification" (EV). The purpose of the IMF is to direct a candidate polypeptide to any one of the antigen processing compartments of an APC where it can be degraded to peptide fragments. Any peptides of the appropriate length (about 9 to 25 amino acid residues), and having specific binding affinity for a particular class II MHC molecule expressed by the APC, will bind to that class II MHC molecule in the antigen processing compartments. The majority of these peptide-class II MHC molecular complexes then migrate to the cell membrane of the APC. The complexes (both cell-membrane associated and intracellular) are isolated from the APC and the peptides eluted from the complexes. The eluted peptides are then separated, their amino acid sequences determined, and the sequences compared to that of the candidate polypeptide.

The IMF can generally be applied to the analysis of peptides produced by an APC expressing defined class II MHC molecules. As such, the method can be useful for basic research studies, e.g., studies aimed at identifying amino acid residues in a polypeptide that determine sites of "cutting" by the proteolytic antigen processing enzymes of APC. Alternatively, where the polypeptide is suspected of being an antigen that activates CD4+ T cells which cause or promote a particular disease or mediate protection from a disease, the IMF can be used to identify disease-related or protective peptide epitopes derived from the polypeptide. This information would be useful for basic research into the etiology of the disease, or as a basis for development of diagnostics, therapeutics, or vaccines for the disease.

A peptide whose amino acid sequence matches that of a region of the candidate polypeptide is likely to be one that activates CD4+ T cells involved in the pathogenesis or immunity to the relevant disease. Such a peptide can be subjected to the EV procedure in which its ability to activate CD4+ T cells from test and control subjects is assayed. Those peptides that activate CD4+ T cells from test subjects but not those from control subjects are identified as peptides that can initiate, promote, or exacerbate the relevant disease or mediate protection from disease or its pathogenic symptoms.

Once such a peptide is identified, it can be synthesized in large amounts, by chemical or recombinant techniques, and used in diagnostic assays similar to the EV procedures listed below. Relevant peptides could be used singly or in combination. Alternatively, expression vectors encoding such a peptide or a combination of such peptides can be used to transfect or transduce appropriate APC (see below), and these can be used in similar diagnostic assays.

Furthermore, multimers (e.g., dimers, trimers, tetramers, pentamers, or hexamers) of a class II MHC molecule containing a peptide defined by the method, if conjugated with a detectable label (e.g., a fluorescent moiety, a radionuclide, or an enzyme that catalyzes a reaction resulting in a product that absorbs or emits light of a defined wavelength) can be used to quantify T cells from a subject (e.g., a human patient) bearing cell surface receptors that are specific for, and therefore will bind, such complexes. Relatively high numbers of such T cells are likely to be diagnostic of a relevant disease or an indication that the T cells are involved in immunity to the disease. In addition, continuous monitoring of the relative numbers of multimer-binding T cells can be useful in establishing the course of a disease or the efficacy of therapy. Such assays have been developed using tetramers of class I MHC molecules containing an HIV-1-derived or an influenza virus-derived peptide [Altman et al. (1996), Science 274:94-96; Ogg et al. (1998), Science 279:2103-2106], and corresponding class II MHC multimers would be expected to be similarly useful. Such complexes could be produced by chemical crosslinking of purified class II MHC molecules assembled in the presence of a peptide of interest or by modification of already established recombinant techniques for the production of class II MHC molecules containing a single defined peptide [Kazono et al. (1994), Nature 369:151-154; Gauthier et al. (1998), Proc. Natl. Acad. Sci. U.S.A. 95:11828-11833]. The class II MHC molecule monomers of such multimers can be native molecules composed of full-length α and β chains. Alternatively, they can be molecules containing either the extracellular domains of the α and β chains or the α and β chain domains that form the "walls" and "floor" of the peptide-binding cleft.

### 1.1 IMF

There are two different IMF methods, IMF-1 and IMF-2.

### 1.1.1 IMF-1

In IMF-1, the APC is contacted with a ligand that has an intrinsic ability to bind to a receptor on the surface of APC. Candidate receptor-ligand pairs are described below. Prior to contacting the APC, the ligand is conjugated with biotin. Where the ligand has been produced by recombinant DNA technology, the biotinylation can be performed in the cells (e.g., bacteria) in which the ligand is generated by methods such as those used to biotinylate the polypeptide antigens used in Example 1. Alternatively, the isolated ligand can be biotinylated in vitro by methods known in the art. The ligand will be conjugated with at least one biotin moiety per molecule. It can have 2, 3, 4, 6 or 10 biotin residues per molecule, provided that it retains the ability to bind to the cell surface receptor.

After binding of the biotinylated ligand (b-L) to its receptor on the cell surface, unbound b-L is removed by washing and the APC is contacted with avidin, a polypeptide that binds to biotin. The avidin can be egg avidin or streptavidin. It can also be recombinant avidin containing at least two biotin-binding domains. Native avidin contains 4 biotin binding domains.

After binding of the avidin to the biotin residue(s) on the b-L bound to the surface of the APC, unbound avidin is removed by washing and the APC is contacted with a polypeptide of interest (PPI), also previously conjugated with biotin. Biotinylation of the PPI can be performed by the same methods described for the ligand and the biotinylated PPI (b-PPI) can contain the same number of biotin residues per molecule. However, in order to avoid possible interference with processing, the PPI will preferably contain 1 biotin moiety per molecule.

After binding of the biotinylated PPI (b-PPI) to the avidin on the APC, unbound b-PPI is removed by washing, and the APC is incubated. While the procedure up till this stage is generally performed on ice (i.e., at about 4°C), the incubation is carried out at 37°C. Alternatively, the incubation may be performed at room temperature (approximately 25°C) or at a temperature between 25°C and 37°C. The incubation may be performed for 30 minutes, 1 hour, 2 hours, 3 hours, 4 hours or 6 hours, depending on which gives optimal recovery of peptides. After the incubation, the class II MHC molecules of interest are isolated by any one of various methods known in the art, e.g., immunoprecipitation. Preferably, it is isolated by affinity chromatography using a described method [Gorga et al. (1987), J. Biol. Chem. 262:16087-16094].

Peptides bound non-covalently to the isolated class II MHC molecules are then eluted from them. A variety of methods known in the art can be used. Preferably, the method will be one described previously [Chicz et al. (1992), Nature 358:764-768] and in Example 1 herein.

The eluted peptides are separated by one of a variety of possible chromatographic methods, e.g., reverse phase chromatography. All the resulting fractions that contain peptides are then individually analyzed by matrix assisted laser desorption in time-of-flight (MALDI-TOF) mass spectrometry, using settings that do not fragment the peptides. The peptides corresponding to all the "peaks" obtained on the MALDI-TOF spectrum can then be subjected to individual amino acid sequence analysis. Alternatively, only those peptides corresponding to peaks that are not observed in a control spectrum generated using a sample of peptides obtained by an identical procedure but omitting the step of contacting the APC with b-PPI, can be subjected to amino acid sequence analysis. The sequences of the individual peptides can be obtained by means known to those in the art. They can, for example, be obtained by MALDI-TOF, using instrument settings resulting in the fragmentation of the peptides into small fragments that are analyzed by the mass spectrometer. The amino acid sequences of the peptides are then compared to that of the PPI. Those with a sequence identical to a region of the PPI are candidates for EV.

Alternatively, instead of determining the amino acid sequences of the eluted peptides, "standard" peptides considered to be possible candidates can be subjected to MALDI-TOF mass spectrometry. A test peptide with a peak at the same position in the spectrum as a standard peptide will likely have the same sequence as the standard peptide.

### 1.1.2 IMF-2

The IMF-2 method is identical to the IMF-1 method except that, instead of contacting the APC with b-L avidin and then with b-PPI, the b-L bound to the APC in IMF-2 is contacted with the PPI conjugated to avidin (av-PPI). Alternatively, the ligand can be conjugated chemically to avidin to give a ligand-avidin complex (L-av). The av-PPI and L-av conjugate can be made chemically by methods known to those in the art. Alternatively, a fusion protein consisting of the PPI and avidin, or of the ligand and avidin, can be made by standard recombinant DNA technology. In either case, the avidin component can be full-length avidin or it can be a fragment of the avidin molecule containing 1, 2, 3, or all 4 biotin-binding domains.

### 1.2 EV

The EV procedure involves testing of peptides identified by IMF for their ability to bind the class II MHC from which they were eluted and activate various CD4+ T cell populations. Peptides with amino acid sequences either identical to those identified by IMF or corresponding to a core sequence derived from a nested group of peptides identified by the IMF are synthesized. The synthetic peptides are then tested for their ability to bind the class II MHC from which they were eluted and activate CD4+ T cells from (a) test subjects expressing the class II MHC molecule of interest and having at least one symptom of the disease; and (b) control subjects expressing the class II MHC molecule of interest and having no symptoms of the disease. Additional control subjects can be those with symptoms of the disease and not expressing the class II MHC molecule of interest. In some diseases (e.g., those with an autoimmune component) responsiveness in the CD4+ T cells of test subjects but not in CD4+ T cells of the control subjects described in (b) provides confirmatory evidence that the relevant peptide is an epitope that activates CD4+ T cells that can initiate, promote, or exacerbate the relevant disease. In other diseases (e.g., cancer or infectious diseases without an autoimmune component), a similar pattern of responsiveness and non-responsiveness to that described in the previous sentence would indicate that the relevant peptide is an epitope that activates CD4+ T cells that can mediate immunity to the disease or, at least, a decrease in the symptoms of the disease.

Absence of a response in subjects with symptoms of the disease but not expressing the class II MHC molecule provides further evidence for the stated activities of the peptide. On the other hand, a response in such CD4+ T cell would not necessarily exclude such a role but would suggest that the relevant peptide is capable of (i) binding to some MHC class II molecule expressed by the relevant subject; and (ii) being recognized by CD4+ T cells in association with that class II MHC molecule.

CD4+ T cell responses can be measured by a variety of *in vitro* methods known in the art. For example, whole peripheral blood mononuclear cells (PBMC) can be cultured with and without a candidate synthetic peptide and their proliferative responses measured by, e.g., incorporation of [³H]-thymidine into their DNA. That the proliferating T cells are CD4+ T cells can be tested by either eliminating CD4+ T cells from the PBMC prior to assay or by adding inhibitory antibodies that bind to the CD4+ molecule on the T cells, thereby inhibiting proliferation of the latter. In both cases, the proliferative response will be inhibited only if CD4+ T cells are the proliferating cells. Alternatively CD4+ T cells can be purified from PBMC and tested for proliferative responses to the peptides in the presence of APC expressing the appropriate class II MHC molecule. Such APC can be B-lymphocytes, monocytes, macrophages, or dendritic cells, or whole PBMC. APC can also be immortalized cell lines derived from B-lymphocytes, monocytes, macrophages, or dendritic cells. The APC can endogenously express the class II MHC molecule of interest or they can express transfected polynucleotides encoding such molecules. Where the subjects are humans, the APC can also be T cells since human T cells are capable of expressing class II MHC molecules. In all cases the APC can, prior to the assay, be rendered non-proliferative by treatment with, e.g., ionizing radiation or mitomycin-C.

As an alternative to measuring cell proliferation, cytokine production by the CD4+ T cells can be measured by procedures known to those in art. Cytokines include, without limitation, interleukin-2 (IL-2), IFN-γ, IL-4, I-5, TNF-α, interleukin-3 (IL-3), interleukin-6 (IL-6), interleukin-10 (IL-10), interleukin-12 (IL-12) and transforming growth factor β (TGFβ) and assays to measure them include, without limitation, ELISA, and bio-assays in which cells responsive to the relevant cytokine are tested for responsiveness (e.g., proliferation) in the presence of a test sample. Alternatively, cytokine production by CD4+ lymphocytes can be directly visualized by intracellular immunofluorescence staining and flow cytometry.

Having identified peptide epitopes that are associated with a particular disease, the EV described above can be used as a diagnostic test for the disease. Thus, lymphocytes from a subject suspected of having or being susceptible to the disease can be tested by any of the described methods for a CD4 T lymphocyte response to one or more (e.g., 2, 3, 4, 5, 6, 10, 15, or 20) appropriate peptides. If a significant CD4 T lymphocyte is detected, it is likely that the subject has or will develop the disease. The disease can be, for example, IDDM and the peptides can be derived from, for example, insulin, proinsulin, preproinsulin, GAD65, IA-2, or phogrin. Appropriate peptides can be, for example, any of those listed below (e.g., those with SEQ ID NOS:1-42).

In addition, peptides identified as being associated with any of the diseases listed herein (e.g., an autoimmune disease such as IDDM, MS, or RA) can be used to induce immunological tolerance in lymphocytes (e.g., CD4+ T lymphocytes) associated with the initiation, progress, or pathological symptoms of the disease. Tolerization of these lymphocytes can be useful for prophylaxis against and/or therapy of the relevant disease. Induction of tolerance can be achieved by administering an appropriate peptide to a subject, e.g., a subject having, suspected of having, or being susceptible to any of the autoimmune diseases described herein, e.g., IDDM, MS, or RA. Methods of testing for efficacy of a peptide in inducing tolerance, methods and routes of administration, and doses to be administered are essentially the same as those described below for APL.

As an alternative to the above-described EV, peptides identified by the IMF can be tested for their ability to bind to an appropriate class II MHC molecule by methods known in the art using, for example, isolated class II MHC molecules or cells transfected with nucleic acid molecules encoding them. One such method is described in Example 2. These binding assays can also be used to test the ability of peptides to bind to alternative class II MHC molecules, i.e., class II MHC molecules other than those from which they were eluted using the IMF method. The diagnostic methods using such peptides and therapeutic methods using either the peptides, can be applied to subjects expressing such alternative class II MHC molecules.

### 1.3 Diseases and their associations with class II MHC genes

The methods can be applied to the analysis of peptides involved in diseases associated with expression of defined class II MHC molecules and in which pathology or protection is due to the action of activated CD4+ T cells. Such diseases include, without limitation, certain infectious diseases, cancer, and autoimmune diseases.

An example of an infectious disease that fulfills the above criteria is human leprosy, which is caused by *Mycobacterium* leprae. The bacteria infect and thrive in peripheral Schwann cells and macrophages. The disease is characterized by repressed cellular immunity but normal antibody responses. Leprosy has been associated with the expression of DRB1 class II MHC molecules in which codon 13 encodes Arg or codons 70 and 71 encode Arg [Zerva et al. (1996), J. Exp. Med. 183: 829-836]. In addition, the ability to spontaneously clear hepatitis C virus is associated with expression of DQ B1*0301 molecules and, since DQB1*0302 is underrepresented in hepatitis virus C infected subjects, DQB1*0302 expressing individuals may be protected form infection with the virus [Cramp et al. (1998), J. Hepatol. 29:207-213]. Furthermore, melanoma cell-specific CD4+ T cells, which may be involved in protective immune responses to malignant melanoma, recognize tyrosinase epitopes-presented by HLA-DRB1*0401 class II molecules [Topalian et al. (1996), supra]. Other MHC class-II associated diseases are listed above.

Examples of autoimmune diseases to which the methods of the invention can be applied include, without limitation, IDDM, rheumatoid arthritis (RA), multiple sclerosis (MS), systemic lupus erythmatosus (SLE), and myasthenia gravis (MG). RA is associated with expression of DRB1 alleles encoding the motifs QKRAA (SEQ ID NO:53), QRRAA (SEQ ID NO:54) or RRRAA (SEQ ID NO:55) at amino residues 70-74 (DRB1*0101, 0401, 0403, 0405). MS is associated with expression of DRB1*1501, DQA1*0102 and DQB1*0602 alleles. SLE is associated with the expression of DRB1*03, DRB1*1501, DQA1*0501 and DQB1*0201 alleles. MG is associated with the expression of DR3 and DQ2 (DQA1*0501-DQB1*0201 and DQA1*0201-DQB1*0201) alleles. Autoimmune ovarian failure is associated with DQB1 genes encoding Asp at position 57. Graves' thyroiditis, Hashimoto's thyroiditis, and primary hypothyroidism all show weak association with the expression of the DR5 and DR3 alleles. Coeliac disease is associated with the expression of HLA-DQA1*0501 and DQB1*0201 alleles. Primary biliary cirrhosis is associated with the expression of DRB1*0801-DQA1-0401/0601-DQB1*04 alleles. Autoimmune hepatitis is associated with the expression of DRB3*0101 or DRB1*0401 alleles. Addison's disease is associated with the expression of DRB1*03, DQA1*0501 and DQB1*0201 alleles. Vitiligo is associated with the expression of DRB1*0701 and DQ2 alleles. Anti-glomerular basement membrane disease (Goodpasture's syndrome) is associated with the expression of DR15 and DR4 alleles.

Pathology in RA, MS, and IDDM is considered to be due predominantly to CD4+ T cell-dependent cell-mediated autoimmune responses, while that of SLE and MG is due predominantly to CD4+ T cell dependent antibody-mediated autoimmune responses. In RA the inflammatory response induced by the activated CD4+ T cells is focused on joint synovia, in MS on neural myelin sheaths, and in IDDM on pancreatic β cells located in the islets of Langerhans. SLE is a systemic autoimmune disease involving multiple organs. The muscle fatigue observed in MG is due to the development in the patient of antibodies that bind to the acetylcholine receptor in neuromuscular junctions.

### 1.3.1 IDDM

Diabetes is a syndrome in which levels of blood glucose are abnormally high. Blood glucose levels are normally controlled by the release of the hormone insulin from β cells located in the islets of Langerhans in the pancreas. Type 1 diabetes (IDDM), the class of diabetes relevant to the present application, results from destruction of β cells. The resulting high blood glucose level, if unchecked, leads to dehydration, acid/base disturbances in the blood, brain swelling, coma and dearth. Treatment with injections of synthetic human insulin restores glucose control. Once initiated, however, this treatment is required for life since β cells do not re-generate. Once established, diabetes is a major burden to the patient, to the patient's family, and to society. Although modern dosages and preparations of insulin can maintain blood glucose within reasonable limits, over several years complications of the disease inevitably occur. The commonest severe complications of diabetes are kidney failure, blindness, and loss of nerve function. In developed countries, diabetes is the single major cause of chronic kidney failure requiring long-term dialysis or transplantation. The life span of a diabetic patient is reduced by an average of 10 years. Being a relatively common disease (IDDM affects 1/200-1/400 of the population), it consumes vast resources; it is estimated that in the developed world the cost of diabetes care is 8% of the acute health services budget.

In light of this background, it is important to consider whether there are ways in which IDDM can be prevented from developing. First, β cell destruction takes place over many months or years until there are too few cells synthesizing insulin (approximately 10% of normal) to sustain normoglycaemia and the patient is diagnosed diabetic. If the process of β cell damage could be halted, IDDM would not develop. Second, it is now possible to predict who will develop IDDM in the future with a high degree of sensitivity (i.e., a good "pick-up" rate) and specificity (a low false positive rate) through simple blood tests. Third, it appears that β cells are destroyed as part of an inadvertent immune response, during which normal' components of the cell (proteins called autoantigens) become the target of an autoimmune attack. Several of the major autoantigens from β cells have been identified (see below). Another key characteristic of IDDM is the strong genetic influence on the development of the disease. Although several genes are involved, the most predominant ones are the class II genes of the human MHC, i.e., the human leukocyte antigen (HLA) genes. These genes exert a dominant control over the immune response, by selecting the peptide segments (epitopes) within autoantigens against which a particular individual's immune system focuses its attack. By identifying these epitopes, it will be possible devise strategies to intervene in the development of the disease at a pre-clinical stage.

The HLA gene complex is the most polymorphic in the human genome, so the possibility of an individual's expressing different HLA molecules is high. However, in patients with IDDM, a very limited set of the class II HLA genes is strongly associated with the development of the disease. As a result, within a racially defined population, particular class II HLA genes are much more common in diabetics compared with the total population. Below are examples of the HLA class II genes found more commonly in North American and North European IDDM patients, and therefore likely to have a strong contributory role to the development of the disease:
Class II HLA-DR types: DRB1*0401, 0405
Class II HLA-DQ types: DQB1*0302, 0201, 0501; DQA1*0501, 0301.

Susceptibility genotypes in Caucasians, Blacks, and Japanese are indicated below:
Caucasians:
   DRB1*04, DQA1*0301, DQB1*0302
   DRB1*04, DQA1*0301, DQB1*0201
   DRB1*03, DQA1*0501, DQB1*0201
Additional susceptibility genotypes in Blacks:
   DRB1*09, DRB1*07, DQA1*0301, DQB1*0201
Additional susceptibility genotypes in Japanese:
   DRB1*08, DQA1*0301, DQB1*0302
   DRB1*09, DQA1*0301, DQB1*0303

A crucial question is: how do HLA class II molecules differ in function between a diabetic possessing HLA- DQA1*0301/DQB1*0302 (DQ8) and a non-diabetic possessing HLA- DQA1*0102/DQB1*0602 (DQ6), which appears to be protective from IDDM? It is known that the different HLA class II molecules select and present different peptide epitopes to T cell receptors. Thus it is probable that a "diabetes promoting" HLA class II molecule selects a peptide epitope in some way that initiates or fosters a dangerous autoimmune response.

In North American and North European IDDM patients, HLA-DRB1*0401 is moderately associated with IDDM, while the DRB1*0405 type has a stronger influence on development of disease. However, a particular group of HLA-DQ molecules contribute the strongest susceptibility. In this group are HLA-DQ molecules that contain an α polypeptide chain with the amino acid arginine at position 52 (arg52α), and a β polypeptide chain with any amino acid other than aspartate at position 57 (non-asp57β). One of the best characterized of these is HLA-DQ8 (see above) which is typically linked to HLA DRB1*0401 (i.e., the two genes are often found together on the same chromosome). These genes confer the highest risk for IDDM [Khalil et al. (1992), Diabetes 41:378-384]. It is estimated that an individual expressing an arg52α/non-asp57β HLA-DQ molecule who has a first degree relative with IDDM has.a 1:4 chance of developing IDDM himself; that is 100 times the population risk [Nepom, G.T. (1995), Annu. Rev. Med. 46:17-25].

### 1.4 Species

The methods can be applied to diseases with the described characteristics in a wide range of mammalian species, e.g., humans, non-human primates, horses, cattle, pigs, sheep, goats, dogs, cats, rabbits, guinea pigs, hamsters, rats, and mice. They will preferably be applied to diseases of humans.

It is known, for example, that certain mouse strains are susceptible to murine forms of RA, MS, IDDM and SLE. Moreover, in these mice, the susceptibility is associated with the expression of particular class II MHC genes and the tissue damage is due to the action of activated CD4+ T cells or CD4+ T cell dependent antibody responses. For example, the non-obese diabetic (NOD) mouse, which is susceptible to spontaneous IDDM, expresses the H-2A^{g7} molecule, and the susceptibility of NOD mice to IDDM has been linked to the H-2A^{g7} gene. Furthermore, the tissue destruction in NOD IDDM has been shown to be mediated by CD4+ T cells. In addition, susceptibility to collagen-induced arthritis (CIA) in mice has been associated with expression of H-2A^{a}, H-2A^{r}, H-2A^{w3}, and H-2A^{w17} class II MHC molecules and the joint pathology in CIA is generally considered to be mediated by CD4+ T lymphocytes.

With respect to cancers, the reticular cell sarcomas of SJL mice are dependent for growth on cytokines produced by activated CD4+ T cells and require the expression of certain class II MHC molecules.

### 1.5 Class II MHC Molecules

Class II MHC molecules have been identified in multiple mammalian species. In some of these species, expression of a particular class II MHC molecule has been associated with a particular CD4+ T cell-mediated diseases (see above). In humans, for example, the class II MHC molecules are designated HLA-DR, HLA-DQ, and HLA-DP and in mice, H-2A and H-2E. In all species, there are multiple alleles of each gene.

### 1.6 Antigen Presenting Cells (APC)

APC that can be used for the IMF methods will be those listed above for use in EV, i.e., B lymphocytes, macrophages, monocytes, dendritic cells, and, in humans, T cells. Alternatively, immortalized lines of such cells can be used.

Ligands that could be used with B lymphocyte APC include lectins such as pokeweed mitogen (PWM); antibodies (or functional fragments of antibodies such as Fab, F(ab')₂ or Fv fragments) that bind to APC surface receptors that are components of the cellular machinery for internalization and presentation of antigen, or are involved in signalling for antigen internalization, e.g., complement receptors (CD21, CD35, CD11b/CD18, CD11c/CD18), the B cell receptor complex (including immunoglobulin molecules), mannose receptors, CD19, CD22, CD40, CD20, and CD45; ligands for the above listed receptors on B cells (e.g., soluble-CD40 ligand) and other APC; and whole Ig molecules of either irrelevant specificity or with the ability to bind to the PPI or a tag (e.g., a peptide or hapten) conjugated to the PPI or fragments of such molecules that include the Fc portion and thus can bind to Fc receptors in APC cell membranes.

Receptors to which the above ligands bind are as follows. PWM, which is derived from *Phytolacca* americana, binds to a number of carbohydrate moieties. It binds selectively to disulfide-linked members of the Ig family of proteins e.g., the surface Ig molecules that constitute the antigen specific receptors of B lymphocytes. Any molecule on the surface of B cells to which PWM can bind will be a receptor for PWM. Lectins that could be used instead of PWM include the following carbohydrate binding molecules: pea lectin, concanavalin A, lentil lectin, phytohemagglutinin (PHA) from Phaseolus vulgaris, peanut agglutinin, soybean agglutinin, Ulex europaeus agglutinin-I, Dolichos biflorus agglutinin, Vicia villosa agglutinin and Sophora japonica agglutinin. The receptors for antibodies or ligands that bind APC surface receptors will, by definition, be the receptors themselves, examples of which are listed above. Receptors for Ig molecules of irrelevant specificity or with the ability to bind to the PPI or a tag conjugated to the PPI, or fragments of such molecules that include Fc portions, are Fc receptors on B lymphocytes, macrophages, and monocytes.

### 1.7 Polypeptide Antigens

Polypeptide antigens that can be used with the IMF methods can be those with a known amino acid sequence or those in which at least part of the amino acid sequence is known. They can be polypeptides that themselves are known or suspected to be involved in the disease process (e.g., IA-2 in IDDM) or they can be derived from microbial organisms known or suspected to be involved in the disease process (e.g., M. leprae in leprosy). Examples of other polypeptide antigens include the core and viral coat proteins of viruses such as hepatitis C virus, the heat shock proteins of mycobacteria, and tyrosinase in melanoma. Furthermore, the polypeptide antigen can be the full-length protein or it can be a fragment of the protein known or suspected to be involved in the disease process (e.g., the intracellular portion of IA-2 in IDDM).

Examples of polypeptides that are suspected autoantigens in MS (including murine experimental autoimmune encephalomyelitis) are myelin basic protein (MBP), proteolipid protein (PLP), myelin oligodendrocyte protein (MOG), and alpha B-crystallin. Collagen is considered to be an autoantigen in RA, the acetylcholine receptor in MG, and Smith protein, RNP ribonucleoprotein, and SS-A and SS-B proteins in SLE. Other autoimmune disease and polypeptides that have been implicated as autoantigens involved in their genesis are listed below:
Autoimmune ovarian failure: 3p hydroxysteroid dehydrogenase
Graves' thyroiditis: thyroglobulin, thyroid peroxidase, and thyroid stimulating hormone receptor
Hashimoto's thyroiditis: thyroglobulin and thyroid peroxidase
Primary hypothyroidism: thyroglobulin and thyroid peroxidase
Coeliac disease: transglutaminase
Primary biliary cirrhosis: pyruvate dehydrogenase Autoimmune hepatitis: cytochrome P4502D6
Addison's disease: 21α hydroxylase
Vitiligo: tyrosinase
Anti-glomerular basement membrane disease (Goodpasture's syndrome): type IV collagen
Systemic sclerosis: Scl-70

A more detailed description of autoantigens suspected to be involved in IDDM is provided below.

The inappropriate autoimmune response that leads to IDDM targets proteins in the pancreatic β cell. There are several autoantigens that have been associated with IDDM, of which 3 are considered to be the major ones: insulin/proinsulin; glutamic acid decarboxylase (65kD isoform; GAD-65); and IA-2. The term "major" here is used to denote the fact that: (a) most (i.e., 80-90%) IDDM patients make an immune response to at least one of these 3; and (b) in the prediction of IDDM in high risk individuals, an immune response to all 3 autoantigens carries a very strong risk of future IDDM.

Insulin is synthesized initially as pre-proinsulin (106 amino acids), and the molecule resulting from cleavage of the leader sequence is designated proinsulin. Proinsulin (82 amino acids) is a single polypeptide looped back upon itself by 2 intra-chain disulfide bonds. The C chain (also called C-peptide, 31 amino acids) of proinsulin is cleaved to give the secreted form of insulin which comprises two chains (A, 30 amino acids long, and B, 21 amino acids long) joined by the 2 disulfide bonds.

Spontaneously arising antibodies to insulin (insulin autoantibodies, IAA) were first identified in untreated newly diagnosed diabetic patients in 1983 [Palmer et al. (1983), Science 222:1337-1339]. Typically, 40-50% of young IDDM or pre-IDDM patients have IAA, while they are rarer in adolescents and adults. Insulin autoantibodies have been shown to react equally well with human, porcine, bovine, rat, sheep and chicken insulin but they fail to react with isolated A or B insulin chains [Castano, L. and Eisenbarth, G.S. (1990), Annu. Rev. Immunol. 8:647-79] suggesting that both chains contribute to form the epitope(s) of these autoantibodies. Recent work has provided more evidence for the involvement of both A and B chains in epitope generation, suggesting that a 6 amino acid sequence in the A chain and a 3 amino acid sequence in the B chain are included in the epitopes recognized by insulin autoantibodies; this region differs from the insulin receptor binding domain [Castano et al. (1993), Diabetes 42:1202-1209]. Autoantibodies to proinsulin can be detected in 22% of prediabetic patients before the onset of type 1 diabetes [Kuglin et al. (1990), Diabet. Med. 7:310-314].

Baekkeskov and co-workers showed that more than 80% of newly diagnosed diabetic children had autoantibodies to a β cell autoantigen of 64 kDa relative molecular mass [Baekkeskov et al. (1982), Nature 298:167-169], and autoantibodies were also present in relatives at high risk of future diabetes onset [Baekkeskov et al. (1987), J. Clin. Invest. 79:926-934; Atkinson et al. (1990), Lancet 335:1357-60]. The molecular identification of the 64 kDa antigen as GAD was made in 1990 [Baekkeskov et al. (1990), Nature 347:151-156]. GAD is an enzyme involved in synthesis of the inhibitory neurotransmitter γ-amino butyric acid and probably has a role in signaling for insulin release. There are two isoforms of the enzyme: GAD65 and GAD67. By far the major representative in human islets of Langerhans is GAD65.
Autoantibodies to GAD65 are present in the serum of 70-80% of patients with new onset IDDM [Petersen et al. (1994), Diabetes 43:459-467]. Like IAA, GAD autoantibodies are an early predictive marker of the disease, associated with high risk for development of IDDM. They are present in over 80% of individuals known to be at high risk of developing IDDM because of a family history and the presence of immune markers [De Aizpurua et al. (1992), Proc. Natl. Acad. Sci. U.S.A. 89:9841-9845; Seissler et al. (1993), J. Clin. Invest. 92:1394-1399].

In early immunoprecipitation experiments, mild trypsin treatment of a 64 kDa islet cell protein resulted in the formation of 40 kDa and 37 kDa protein fragments which bind autoantibodies present in the sera of patients with IDDM [Christie et al. (1990), J. Exp. Med. 172:789-794]. Most importantly, autoantibodies to these fragments were shown to be highly predictive of IDDM in at-risk individuals [Christie et al. (1994), Diabetes 43:1254-1259]. The molecular targets of these autoantibodies have now been identified. The 40 kDa fragment is a component of IA-2, also confusingly called ICA512 [Payton et al. (1995), J. Clin. Invest. 96:1506-1511; Bonifacio et al. (1995), J. Immunol. 155:5419-5426; and Rabin et al. (1994), J. Immunol. 152:3183-3188]. Subsequently, the 37 kDa fragment was identified as phogrin (IA-2β), a tyrosine phosphatase which shares 85% homology with IA-2. Autoantibodies to IA-2 and phogrin appear during the prediabetic period [Bonifacio et al. (1998), J. Immunol. 161:2648-2654] and are highly predictive of IDDM development in at-risk individuals. IA-2 is synthesized as a large protein of 106 kDa which has an intracellular domain at residues 603-1055. The intracellular domain of IA-2 is the target of almost all autoantibody reactivity to IA-2 [Kawasaki et al. (1997), J. Clin. Endocrinol. Metab. 82:375-80].

### 2. Peptides

Peptides of the invention are derived from IA-2 and can bind to HLA-DR4 molecules. The peptides can be, for example, any one of the following peptides: YLKNVQTQETRTL (SEQ ID NO:10); YLKNVQTQETRTLTQ (SEQ ID NO:13); FYLKNVQTQETRTLTQFHF (SEQ ID NO:16); AYQAEPNTCATAQ (SEQ ID NO:17); LAKEWQALCAYQAEPNT (SEQ ID NO:19); AYQAEPNTCATAQGEGNIK (SEQ ID NO:20); WQALCAYQAEPNTCATAQ (SEQ ID NO:21); LAKEWQALCAYQAEPNTCATAQGE (SEQ ID NO:22); and KLKVESSPSRSDYINAS (SEQ ID NO:40)

The peptides can be prepared using the described IMF methodologies. Smaller peptides (less than 50 amino acids long) can also.be conveniently synthesized by standard chemical means. In addition, both polypeptides and peptides can be produced by standard *in vitro* recombinant DNA techniques, synthetic techniques, and *in vivo* recombination/genetic recombination, using the nucleotide sequences encoding the appropriate polypeptides or peptides. Methods well known to those skilled in the art can be used to construct expression vectors containing relevant coding sequences and appropriate transcriptional/translational control signals. See, for example, the techniques described in Maniatis et al., Molecular Cloning: A Laboratory Manual [Cold Spring Harbor Laboratory, N.Y., 1989], and Ausubel et al., Current Protocols in Molecular Biology, [Green Publishing Associates and Wiley Interscience, N.Y., 1989].

A variety of host-expression vector systems can be used to express the peptides and polypeptides. Such host-expression systems represent vehicles by which the polypeptides of interest can be produced and subsequently purified, but also represent cells that can, when transformed or transfected with the appropriate nucleotide coding sequences, produce the relevant peptide or polypeptide in situ. These include, but are not limited to, microorganisms such as bacteria, e.g., *E. coli* or *B. subtilis,* transformed with recombinant bacteriophage DNA, plasmid or cosmid DNA expression vectors containing TR₁₋₄₁ peptide coding sequences; yeast, e.g., *Saccharomyces* or Pichia, transformed with recombinant yeast expression vectors containing the.appropriate coding sequences; insect cell systems infected with recombinant virus expression vectors, e.g., baculovirus; plant cell systems infected with recombinant virus expression vectors, e.g., cauliflower mosaic virus (CaMV) or tobacco mosaic virus (TMV), or transformed with recombinant plasmid expression vectors, e.g., Ti plasmids, containing the appropriate coding sequences; or mammalian cell systems, e.g., COS, CHO, BHK, 293 or 3T3, harboring recombinant expression constructs containing promoters derived from the genome of mammalian cells, e.g.; metallothionein promoter, or from mammalian viruses, e.g., the adenovirus late promoter or the vaccinia virus 7.5K promoter.

The following examples are meant to illustrate, not limit, the invention.

### EXAMPLES

### Materials and Methods

Culture of Epstein-Barr Virus (EBV) Transformed B Cell Lines. EBV transformed B lymphocyte lines were propagated in RPMI 1640 medium supplemented with glutamine, penicillin/ streptomycin and 10% fetal calf serum (FCS) in 50-100 175 cm² flasks to achieve high volumes of cells. The EBV transformed cells used were Priess cells which are homozygous for the IDDM-permissive DRB1*0401, DRB4*0101 [DR4/DRw53], DQA1*0301/DQB1*0302[DQ8] HLA genotype.
Approximately 50% of cells were harvested every 2-3 days by pelleting, washed with Hanks balanced salts solution (HBSS), counted, resuspended in HBSS, and used for the IMF procedure.

Biotinylated Polypeptide Antigens. Recombinant antigens were generated in *E*. *coli.* The intracellular portion of IA-2 (IA-2ic) was generated using the Pinpoint Vector (Promega, Madison, WI) which produces fusion proteins coupled at the N-terminus to a leader sequence biotinylated at a single lysine residue. This permitted purification using monomeric avidin columns, and also produced a biotinylated form of the antigen of interest for use in the Antigen Delivery System (ADS) (see below). The Pinpoint vector containing cDNA encoding IA-2ic was kindly provided by Dr. M. Christie, King's College London [Payton et al. (1995), J. Clin. Invest. 96:1506-1511]. The conditions for the purification of IA-2ic were as previously established [Payton et al. (1995), supra]. In brief, E. coli strain JM109 cells were transformed with the Pinpoint vector containing the IA-2ic cDNA.

Colonies were subcultured onto minimal media/agarose plates and single colonies picked and cultured overnight at 37°C with shaking in minimal media containing 2 µM d-biotin and 100 µg/ml ampicillin. Once the culture had attained an A₆₀₀ of 0.5, it was transferred (1:10 dilution) into LB medium containing 2 µM d-biotin and cultured at 37°C with shaking for one hour. Protein expression was induced in the logarithmic phase of growth using 100 µM isopropyl β-D-thiogalactopyranoside (IPTG).
Cells were harvested after 3-5 hours shaking at 37°C, by centrifuging at 8,000g at 4°C. The cell pellet was resuspended in cell pellet buffer (CPB; 100 mM phosphate buffer, pH 7.2 containing 10 mM benzamidine and 1 mM phenylmethylsulfonylfluoride).

Cells were then lysed on ice and soluble proteins released using a combination of lysozyme (1 mg/ml), Triton X-100 (0.1%) and deoxyribonuclease (200 U/ml) treatment. After removal of cell debris by centrifugation (14,000g for 15 minutes at 4°C), the biotinylated fusion protein was purified from the supernatant by passage, at a flow rate of 8 ml/hour, over an avidin-resin column (SoftLink, Promega, Madison, WI) prepared according to the manufacturer's instructions and equilibrated in CPB. After extensive column washing, the biotinylated fusion protein was eluted using an excess of 5 µM d-biotin, separated from free d-biotin using a G-25 column (Pharmacia), and concentrated 10- to 100-fold using an Amicon B15 concentrator with a 15 kDa molecular weight "cutoff." Purity, which was typically >90%, was assessed by SDS-PAGE and Western blot analysis in which avidin-peroxidase was used in the developing step.

GAD65 cDNA obtained from RNA extracted from human pancreatic islets was cloned into the pET 12 vector (Stratagene.), in which expression is controlled by the T7 promoter downstream of a biotinylation tag sequence and a histidine purification tag designed based on the Pinpoint vector. This pET 12 vector system has the advantage that fusion protein expression can be induced in the protease deficient strain of E. coli, BLR (DE3) pLysS.

GAD65 was generated as follows. BLR (DE3) pLysS bacteria were transformed with the GAD65 cDNA containing vector and a colony picked into LB and grown at 37 °C with shaking at 225 rpm until an A₆₀₀ of 0.6-1.0 was reached. Cells were then resuspended in fresh LB, seeded at a dilution of 1:25, grown under the same conditions to an A₆₀₀ 0.4, and induced at 30°c with 2 mM IPTG for 3 hours. A bacterial pellet obtained by centrifugation was resuspended in 8 M guanidine hydrochloride (GuHCl), 50 mM NaH₂PO₄, 10 mM Tris, 0.1% Triton X-100, 50 mM 2-mercaptoethanol (2-ME), pH 8.0; sonicated; and centrifuged for 1 hour at 4°C at 40,000g. The supernatant was dialyzed against a 10x excess of the 8M GuHCl buffer without 2-ME and then added to a 50% nickel resin slurry for 1 hour, rocking at room temperature. The nickel resin was resuspended in a column and washed with urea buffers provided by the manufacturers of the nickel resin (Qiagen, Germany) but supplemented with 5mM 2-ME and 0.1% Triton X-100. Proteins were eluted using urea buffers of pH 5.9 and pH 4.5 and dialyzed against 4M urea, containing 50 µM pyridoxal phosphate, 20 mM sodium glutamate, 0.05% Triton X-100, 5 mM 2-ME and 2M L-arginine. The preparation was then dialyzed against sodium dodecylsulfate (SDS) (0.1%) gel running buffer containing 2.5 mM glutathione, 50 µM pyridoxal phosphate. Dialysis was repeated against an identical buffer containing a 10-fold lower concentration of SDS. Dialysis was then performed against a solution containing 4 mM hepes, 20 mM sodium glutamate, 50 µM pyridoxal phosphate, 2.5 mM glutathione. Final dialysis was against the same buffer without sodium glutamate. At this stage, the yellow, biotinylated GAD65 was stored at 4°C or lyophilized.

Human pre-proinsulin cDNA was kindly provided by Dr. D. Steiner and has been cloned as described above for GAD65. Biotinylated pre-proinsulin was produced and purified under conditions similar to those for production and purification of GAD65.

Antigen delivery system (ADS). For the ADS, harvested and washed Priess cells were suspended at 5 x 10⁷/ml in cold HBSS supplemented with b-PMW (300ng/ml) and incubated on ice for 30 mins. After washing in HBSS, cells were resuspended at 5 x 10⁷/ml in HBSS containing 0.5 mg/ml avidin and incubated on ice for 30 mins. After washing, the cells were resuspended in HBSS supplemented with 10-40 µg/ml biotinylated IA-2ic and incubated for 30 mins on ice. After washing, the cells were resuspended in pre-warmed RPMI 1640/10% FCS (1 x 10⁶/ml) and cultured at 37°C in 5% CO₂ for 6 hours. The cells were pelleted and stored at -80°C until HLA molecule purification was performed.

HLA class II_purification. DR4 molecule purification was carried out as previously described [Gorga et al. (1987), J. Biol. Chem. 262:16087-16094). Cell pellets that had been obtained from the ADS and stored at -80°C were thawed and homogenized in hypotonic buffer. A crude membrane fraction was prepared by high-speed centrifugation and solubilized in NP40. The detergent-soluble fraction was passed over a series of immunoaffinity columns containing Protein A-Sepharose or AffiGel 10 matrix material conjugated with monoclonal antibodies (mAb) that bind to MHC class I molecules (mAb W6/32), DR molecules (mAb LB3.1 or mAb L243), and DQ3 family molecules (mAb IVD12), respectively. Each of these mAbs recognizes the native dimer conformation of the HLA class I or class II molecules on cells of the indicated B lymphocyte lines. The immunoaffinity columns were eluted with 50 mM glycine, pH 11.5/0.1 % sodium deoxycholate, and immediately neutralized and dialyzed against 10 mM Tris, pH 8.0/0.1 % sodium deoxycholate. Protein purity was assessed by SDS-PAGE and quantitated by the BCA assay.

Peptide Analysis. All HLA class II protein samples were concentrated to 100 µl using an ultrafiltration device (Amicon Centricon 10) prior to peptide extraction. Naturally processed peptide repertoires were acid eluted from HLA class II molecules by adding 800 µl 10 % acetic acid, and incubated for 15 minutes at 70°C, as described [Chicz et al. (1993), J. Exp. Med. 178:24-47]. The peptides were separated from the remaining HLA protein by ultrafiltration with the Centricon 10 device. The "flow-through" fraction containing the acid-extracted peptides was concentrated on a Savant SpeedVac to a volume of approximately 20-30 µl and stored at -80°C. The acid-extracted peptide mixtures were then separated by reverse phase chromatography as previously described [Chicz et al. (1993), supra], but with minor modifications. Briefly, the separations were carried out using a microbore C18 column (1.0 x 250 mm; Vydac, Hesperia, CA) with a flow rate of 50 pl/minute. The column effluent was split such that 2% was immediately loaded onto a matrix assisted laser desorption in-time-of-flight (MALDI-TOF) mass spectrometry sample plate, with the remaining 98% being collected for storage at -20°C. The samples were prepared for mass spectrometry analysis by adding 0.4 µL of matrix (α-cyano-4-hydroxycinnamic acid, 10 mg/ml in 50% acetonitrile/0.1 % trifluoroacetic acid) and allowed to air dry. Mass spectra were collected at optimum laser intensities by averaging the ion signals from 128 individual scans in both linear and reflector modes using a single stage extended length reflector time-of-flight mass spectrometer (Voyager Elite XL; PerSeptive Biosystems, Framingham, MA). Time to mass conversion was performed by external calibration using synthetic peptides.

An automated microcapillary liquid chromatography-mass spectroscopy (LC-MS) approach with data dependent collision-assisted dissociation (CAD) for sequencing low levels of naturally processed HLA associated peptides was developed to directly sequence targeted peptide masses as determined by the MALDI-TOF-MS approach previously described. Peptide fractions separated by reversed phase chromatography are diluted to a final volume of 5-20 µl to aid handling and permit the use of second dimension reversed phase separations. The resultant peptide solution can then be preconcentrated by trapping peptides using a small bed (0.5 - 1.0 µL) of polymeric reversed phase support. This also facilitates removal of hydrophilic contaminants by washing the trap with an aqueous solution. Subsequently, peptides are back flushed from the trapping phase onto the microcapillary (with an inner diameter of 75 pm and packed with 5-15 cm of 1-7 µm 100-200 Å C₁₈ or nonporous material) and separation is developed using a non-linear gradient. A mobile phase flow rate of -0.5 µL/min is achieved by splitting the flow from the pumps and using a balance column. Peptide detection is by p-electrospray MS. The voltage necessary to drive the electrospray is applied at the head of the microcapillary column and peptides are electrosprayed into the mass analyzer directly as they elute from the capillary. CAD experiments are triggered in a data dependent mode, using ions that are more abundant than a user-set threshold. Dynamic exclusion is used to ensure maximum peptide coverage (i.e., minor responses are analyzed by CAD following a user determined number of CAD experiments of a single peptide response) by writing an exclusion list during assay progression so that a given ion will not be analyzed by multiple CAD experiments. The time that a given ion resides on the exclusion list is dependent upon the quality of the chromatographic separations. This must be determined experimentally. In this way, separated isobaric responses may be analyzed. Peptide sequencing sensitivity better than 1 fmol can be achieved using this method.

### Epitope Verification (EV).

To establish that peptide epitopes identified are relevant to IDDM (i.e., that they are recognized by CD4+ T cells of patients with IDDM or pre-IDDM expressing the DR4 molecule but not by non-diabetic controls also expressing the DR4 molecule), T cell proliferation assays were carried out using synthetic peptides having amino acid sequences based upon the peptides identified by mass spectrometry to be derived from IA-2ic. Peptides were synthesized using Fmoc chemistry with an Applied Biosystems SYNERGY peptide synthesizer and purified by preparative RP-HPLC on a Waters 2690 Alliance system equipped with a Radial Compression Module. The amino acid sequences and purity of greater than 90% for all the synthetic peptides was confirmed by MALDI-MS and analytical HPLC. Peripheral blood mononuclear cells from recent onset IDDM patients (<6 months from diagnosis) and healthy controls expressing the appropriate HLA DR4 molecules were separated by density gradient centrifugation and co-cultured in wells of 96-well U-bottom plates with peptides at a concentration of 10 µg/ml for 5 days in 150 µl RPMI 1640/10% pooled normal AB serum, followed by pulsing with 0.5 µCi [³H]-thymidine/well and harvesting onto filters for radioactivity counting measured in counts per minute (cpm). There were twelve replicate wells per test group. Results were expressed as a stimulation index (SI) which is the ratio of the cpm obtained from cultures containing peptide to the cpm obtained from cultures without peptide (mean cpm of 12 wells in each case).

The data were also analyzed in terms of the fraction of "positive culture wells." A positive culture well was one that contained peptide and resulted in cpm > mean cpm + 2SD obtained from cultures without peptide. T cell responses were considered significant when the SI is >2.0 and >40% wells are positive.

### Binding Assay:

Synthetic peptides with amino acid sequences based on the 6 core regions identified by the IMF were tested for their ability to bind to isolated HLA-DR4 molecules in a binding inhibition assay performed essentially as previously described [Chicz et al. (1997), J. Immunol. 159: 4935-4942]. In brief, aliquots of immunopurified preparation of HLA-DR4 (final concentration of 10 µg/ml) were incubated with a biotinylated HLA-DR4 binding peptide (consisting of residues 98-117 of class II MHC invariant chain) ("the indicator peptide") (1 µM) and varying concentrations of the test peptides in 0.2 ml tubes. After an overnight incubation at room temperature, the contents of each tube were transferred to a well of a 96-well plastic microtiter plate precoated with anti-HLA-DR4 antibody. The microtiter plates were rocked for 60 min at room temperature and unbound material was removed by rigorous washing. The relative amount of bound standard peptide in each well was determined by measuring color development after addition of streptavidin-conjugated alkaline phosphatase, washing, and adding a chromogenic alkaline phosphatase substrate.

### Example 1. Analysis of HLA DR4 binding Peptides Derived by Natural Processing of IA-2ic By B Lymphocytes

To establish whether the described ADS leads to the generation of peptides (bound to HLA class II molecules on the surface) similar to those produced by APC following natural uptake of a parent polypeptide, a tetanus toxoid- (TT-) specific CD4+ T cell line (NG2) was generated. NG2 cells showed similar high levels of [³H]-thymidine incorporation when co-cultured with APC in which biotinylated TT had been directed to the antigen processing organelles using the described ADS (mean cpm=7750 after 3 days of culture) as when cultured with normal APC and TT (mean cpm=8427). The background value obtained using APC without TT was 2528 cpm. After performing the ADS, aliquots of the Priess cells were incubated at 37°C for 0, 1, 3, or 6 hours and then tested for the presence of TT on their surfaces by sequential treatment with rabbit anti-TT ("anti-TT") antiserum and FITC goat anti-rabbit Ig ("FARIG"), followed by flow cytometry analysis (Fig. 1). Compared with background samples treated with FARIG and not anti-TT (-), surface expression of TT was high at 0 hours (●●●●), had diminished by 1 (-●- ) and 3 (- - -) hours, and was completely absent by 6 hours (● ● ●). This experiment showed that proteins delivered via the ADS are internalized rapidly and are directed into the HLA class II antigen processing pathway, and that relevant peptide epitopes are presented to responsive CD4+ T lymphocytes.

The islet autoantigen IA-2ic was targeted onto the surface of Priess EBV-transformed B lymphocytes using the antigen delivery system (ADS) described above. In the first step, 5-10 x 10⁷ Priess EBV transformed B cells were incubated with b-PWM. After washing away unbound b-PWM, avidin was added to the cell suspension to provide a bridge between the b-PWM and the b-IA-2ic.

After pulsing with the biotinylated IA-2ic, the cells were incubated for 1-6 hours at 37°C to allow internalization, processing and presentation. A control population of cells was pulsed with b-PWM and avidin only. HLA-DR4 (0401) molecules were purified from each cell pellet, bound peptides were eluted and separated by RP-HPLC, and each of 100 fractions was analyzed by MALDI-TOF. RP-HPLC analysis was highly reproducible, with chromatographic traces from the IA-2ic-pulsed and control HLA-DR4 preparations showing a similarity index of 96-99%. A subtractive approach was used to identify IA-2ic-derived peptides. Mass spectra of equivalent RP-HPLC fractions from biotinylated IA-2ic-pulsed and control preparations were overlaid and masses common to both were discounted from further analysis. An example of such a profile is shown in Fig. 2. The mass spectra for the HLA-DR4 (0401) peptide repertoire isolated from Priess cells pulsed with IA-2ic were compared to the spectra for the peptide repertoire isolated from control Priess cells to identify novel m/z (mass to charge ratio) values corresponding to peptides derived from IA-2ic (Fig. 2). In Fig. 2, while peaks with m/z values of about 1747 and 1822 were seen in the spectra obtained with peptide mixtures from both IA-2ic-pulsed and control Priess cells, peaks with m/z values of 1779.75 and 1935.8 were seen only in the spectrum obtained with the peptide mixture from IA-2ic pulsed Priess cells.

The experiment was performed in triplicate. Of the approximately 3000 m/z values observed, 85 novel masses were initially identified as potential naturally processed peptides from IA-2ic. Subsequent mass analyses using higher resolution and more stringent mass accuracy revealed 24 m/z values to have masses corresponding to candidate synthetic peptides derived from IA-2ic. These synthetic peptides were subjected to the mass spectrometry analysis. The mass identification was highly reproducible, with the same 24 masses being identified in three separate B cell preparations and 3 separate RP-HPLC separations. The same masses were seen when B cells were allowed to internalize, process and present antigen for 1 hour and 6 hours, although better peptide loading of DR4 molecules was seen at 1 hour. The sequences of the masses are shown in Table 1. Each of the sequences was a member of one of 6 nested sets of peptides. Nested sets are groups of peptides based around the same core region, but variably truncated or extended at the N- and C-termini. All 6 core regions contained amino acids known to be preferred for HLA-DR4 (0401) binding. The sequences of peptides with SEQ ID NO:10, SEQ ID NO:13, and SEQ ID NO:25 have been confirmed using the above-described CAD methodology applied to samples of the relevant MALDI-TOF separated material. Partial sequences corresponding to several peptides from each of the core regions previously described have also been obtained.

**Table 1. Experimentally observed and calculated masses of IA-2 derived peptides eluted from HLA-DR4 (0401).**

| observed m/z | Calculated m/z | Residues | Corresponding Iλ-21α sequence | Synthetic peptide used in Primary T cell assay |
|---|---|---|---|---|
| 1469.31 | 1468.65 | 657-671 | VSSQFSDAAQASPSS (SBQ ID NO:1) | 654-674 VSSVSSQFSDAAQASPSSHSS (SEQ ID NO:18) |
| 1469.31 | 1468.65 | 656-670 | SVSSQFSDAAQASPS (SEQ ID NO:2) | |
| 1469.31 | 1468.65 | 655-669 | SSVSSQFSDAAQASP (SEQ ID NO:3) | |
| 1866.76 | 1866.80 | 656-674 | SVSSQFSDAAQASPSSHSS (SEQ ID NO:4) | |
| 2397.16 | 2397.08 | 652-675 | SRVSSVSSQFSDAAQASPSSHSST (SEQ ID NO:5) | |
| 2441.48 | 2441.02 | 656-679 | SVSSQFSDAAQASPSSHSSTPSWC (SEQ ID NO:6) | |
| 2485.29 | 2483.03 | 657-680 | VSSQFSDAAQASPSHSSTPSWCE (SEQ ID NO:7) | |
| 1367.44 | 1367.57 | 718-730 | AYQAEPNTCATAQ (SEQ ID NO:17) | 709-732 LAKKWQALCAYQABPNTCATAQGE (SEQ ID NO:22) |
| 1640.86 | 1640.68 | 716-731 | LCAYQAEPNTCATAQG (SRO ID NO:18) | |
| 1935.92 | 1935.91 | 709-725 | LAKEWQALCAYQAEPNT (SEQ ID NO:19) | |
| 1965.83 | 1965.88 | 718-736 | AYQAEPNTCATAQGEGNIK (SEQ ID NO:20) | |
| 1968.75 | 1968.84 | 713-730 | WQALCAYQAEPNTCATAQ (SEQ ID NO:21) | |
| 1489.64 | 1489.15 | 802-815 | GCTVIVMLTPLVED (SEQ ID NO:23) | 797-817 NVWESGCTVIVMLTPLVEDGV (SEQ ID NO:32) |
| 1489.64 | 1489.75 | 803-816 | CTVIVMLTPLVEDG (SEQ ID NO:24) | |
| 1762.88 | 1762.85 | 800-816 | ESGCTVIVMLTPLVEDG (SEQ ID NO:25) | |
| 1779.85 | 1780.19 | 797-812 | MVWESGCTVIVMLTPL (SEQ ID NO:26) | |
| 1861.16 | 1860.97 | 801-818 | SGCTVIVMLTPLVEDGVK (SEQ ID NO:27) | |
| 1861.16 | 1860.97 | 800-817 | ESGCTVIVMLTPLVRDGV (SEQ ID NO.28) | |
| 1883.44 | 1882.88 | 795-810 | WQMVWESGCTVIVMLT (SEQ ID NO:29) | |
| 2144.95 | 2144.97 | 793-810 | DFWQMVWESGCTVIVMLT (SEQ ID NO:30) | |
| 2341.17 | 2339.15 | 794-813 | FWQMVWESGCTVIVMLTPLV (SEQ ID NO:31) | |
| 1508.65 | 1508.74 | 861-872 | TQETRTLTQFHF (SEQ ID NO:9) | 854-872 FYLKNVQTQETRTLTQFHF (SEQ ID NO:16) |
| 1539.34 | 1593.85 | 855-867 | YLKNVQTQETRTL (SEQ ID NO:10) | |
| 1735.78 | 1735.87 | 859-872 | VQTQETRTLTQFHF (SEQ ID NO:11) | |
| 1806.79 | 1806.96 | 856-870 | LKNVQTQETRTLTQP (SEQ ID NO:12) | |
| 1822.41 | 1822.96 | 855-869 | YLKNVQTQETRTLTQ. (SEQ ID NO:13) | |
| 1831.87 | 1830.94 | 851-871 | KNVQTQETRTLTQFH (SEQ ID NO:14) | |
| 2341.17 | 2341.19 | 853-871 | SFYLKVQTQSTRTLTQFH (SEQ ID NO:15) | |
| 1469.31 | 1469.67 | 957-969 | DQFEFALTAVAEE (SEQ ID NO:33) | 955-975 SKDQFEFALTAVAEEVNAILK (SEQ ID NO:37) |
| 1866.76 | 1866.90 | 957-973 | DQFEFALTAVAEEVNAI (SEQ ID NO:34) | |
| 1935.49 | 1936.04 | 959-976 | FEFALTAVAEEVNAILKA (SEQ ID NO:35) | |
| 1968.75 | 1968.95 | 955-972 | SKDQFEFALTAVAEEVNA (SEQ ID NO:36) | |
| 1367.44 | 1367.67 | 754-765 | KVESSPSRSDYI (SEQ ID NO:38) | 753-771 LKVESSPSRSOYINASPII (SEQ ID NO:42) |
| 1367.44 | 1367.67 | 753-764 | LKYESSPSRSDY (SEQ ID NO: 39) | |
| 1880.98 | 1880.96 | 752-768 | KLKVESSPSRSDYINAS (SEQ ID NO:40) | |
| 2441.48 | 2441.19 | 754-775 | KVESSPSRSDYINASPIISHDP (SEQ ID NO:41) | |
| | | | LKVESSPSRSDYINASPII (SEQ ID NO:42) | |

Six synthetic peptides with amino acid sequences based on the 6 core regions of IA-2ic were used to examine peripheral blood T cell responses in IDDM patients (expressing and not expressing HLA-DR4) and in healthy control subjects expressing HLA-DR4 (Table 2). Of 13 HLA-DR4 IDDM patients, 9 had T cells that showed significant ("POS" in Table 2) proliferative responses to at least one of the 6 peptides. Eleven of the DR4 patients expressed the 0401 allele and two expressed both the 0403 and 0405 alleles. The 0401, 0403, and 0405 genes encode similar DRβ chains, differing only at positions 57 (0405 S for D), 71 (0403 and 0405 R for K), 74 (0403 E for A) and 86 (0403 V for G) [Marsh, S.G., Tissue Antigens 51:467-507, (1998)]. The peptide binding motifs of these HLA-DR4 types are known and are similar, and all are predicted to bind to the 6 IA-2ic core peptide regions. T cells from only 1/8 non-DR4 IDDM patients proliferated when exposed to any of the peptides, and none of those from the control subjects (all 0401) responded to any of the peptides. Peptides from all 6 of the 6 core regions elicited T cell responses, and T cells from most responder patients proliferated to a single peptide.

*In toto*, these data indicate that IMF method applied to the analysis of peptides produced by natural processing of IA-2ic resulted in the characterization of peptides that are recognized by CD4+ T lymphocytes specifically from HLA DR4 expressing IDDM patients and thus may be implicated in the IDDM disease process. This finding represents a significant advance in knowledge regarding the aetiology of IDDM and provides the basis for the development of therapeutic and/or prophylactic agents for IDDM, e.g., APL. It is expected that analogous methodologies can be similarly successful in identifying peptides involved in the CD4+ T lymphocyte-mediated pathogenesis of other diseases (see above) in which susceptibility is linked to the expression of a particular class II MHC molecule.

**Table 2. Responses of T cells from patients with IDDM and control subjects to eluted 1A-2 peptides**

| Case | Age (years) | Duration (weeks) | *DRB1* genotype | IA-2 anto-antibodies | T cell response to IA-2 peptide | | | | |
|---|---|---|---|---|---|---|---|---|---|
| | | | | | 654-674 | 709-732 | 955-975 | 797-817 | 854-872 |
| HLA-DR4 | | | | | | | | | |
| IDDM patients | | | | | | | | | |
| S(G) | 17 | 8 | *0401,0101* | + | | | | POS | |
| G(G) | 26 | 12 | *0401,1302* | + | POS | | | | |
| K(G) | 28 | 28 | *0401,1101* | + | POS | | | | |
| ML | 29 | 3 | *0401,0403* | - | | | | | POS |
| EW(B) | 29 | 16 | *0401*/*0401* | + | | | | POS | |
| RW(B) | 20 | 4 | *0401*/*0401* | + | POS | | | | |
| TH(B) | 19 | 4 | *0401*/*0301* | - | | | POS | | |
| DC(I) | 6 | 4 | *0403,0405* | + | | POS | | | |
| GR | 13 | <1 | *0403,0405* | + | POS | | | POS | POS |
| NC(B) | 36 | 16 | *0401*/*0404* | + | | | | | |
| HW | 15 | 12 | *0401*/*0401* | | | | | | |
| LG(G) | 16 | 4 | *0401,0301* | + | | | | | |
| RM | 24 | 1 | *0401,1302* | | | | | | |

| IDDM patients | | | | | | | | | |
|---|---|---|---|---|---|---|---|---|---|
| (non-DR4) | | | | | | | | | |
| ID | 28 | 4 | *0102,0301* | - | POS | | | POS | POS |
| PQ | 20 | 25 | *0301* | - | | | | | |
| MI | 16 | 12 | *1201,1301* | + | | | | | |
| ML(I) | 10 | 12 | *0101,1101* | - | | | | | |
| ST (I) | 13 | 2 | *0301,1301* | + | | | | | |
| OA | 23 | 1 | *1101,1301* | - | | | | | |
| RM | 24 | 25 | *0301,0901* | - | | | | | |
| JH(B) | 33 | 20 | *0301*/*08* | - | | | | | |

| HLA-DR4 | | | | | | | | | |
|---|---|---|---|---|---|---|---|---|---|
| Controls | | | | | | | | | |
| TL(G) | 36 | - | *0401,0101* | - | | | | | |
| JB | 17 | - | *0401*/*0101* | - | | | | | |
| B(G) | 30 | - | *0401,1302* | - | | | | | |
| MR | 24 | - | *0401,1501* | - | | | | | |
| PH | 40 | - | *0401,14* | - | | | | | |
| VB | 16 | - | *0401,0403* | - | | | | | |
| AZ | 24 | - | *0401,02* | - | | | | | |
| CF(G) | 30 | - | *0401,0701* | - | | | | | |

### Example 2. Binding of consensus peptides to isolated HLA-DR4 molecules

In order to test for the ability of the 6 consensus peptides representing the 6 core regions defined by the IMF described in Example 1, a binding inhibition assay was performed (Fig. 3). R1 was the peptide consisting of residues 797-817 of IA-2; R2 was the peptide consisting of residues 854-872 of IA-2; R3 was the peptide consisting of residues 753-771 of IA-2; R4 was the peptide consisting of residues 654-674 of IA-2; R5 was the peptide consisting of residues 709-732 of IA-2; R6 was the peptide consisting of residues 955-975 of IA-2; and Ii-c was the same as the indicator peptide (i.e., a peptide consisting of residues 98-117 of class II MHC invariant chain). The data presented in Fig. 3 indicate that: R4 and R5 bind strongly to HLA-DR4 molecules; Ii-c, R1, and R6 bind with intermediate avidity; and R2 and R3 bind weakly. These findings confirm that, as predicted by the IMF and EV procedures described in Example 1, the six consensus peptides (R1, R2, R3, and R4-R6) all bind to DR4 molecules. Thus, this type of binding assay or others known in the art (e.g., direct binding rather than binding inhibition assays) can be used as additional or substitute EV procedures to that described in Example 1.

Although the invention has been described with reference to the presently preferred embodiment, it should be understood that various modifications can be made without departing from the the invention. Accordingly, the invention is limited only by the claims which follow.

Preferred embodiments according to the invention are as follows:

## Claims

1. An isolated peptide that binds to HLA-DR4, consisting of the amino acid sequence:
(1) YLKNVQTQETRTL (SEQ ID NO: 10);
(2) YLKNVQTQETRTLTQ (SEQ ID NO: 13); or
(3) FYLKNVQTQETRTLTQFHF (SEQ ID NO: 16).

2. The isolated peptide of claim 1, consisting of the amino acid sequence YLKNVQTQETRTL (SEQ ID NO: 10).

3. The isolated peptide of claim 1, consisting of the amino acid sequence YLKNVQTQETRTLTQ (SEQ ID NO: 13).

4. The isolated peptide of claim 1, consisting of the amino acid sequence FYLKNVQTQETRTLTQFHF (SEQ ID NO: 16).

5. An isolated peptide that binds to HLA-DR4, consisting of the amino acid sequence:
(1) AYQAEPNTCATAQ (SEQ ID NO: 17);
(2) LAKEWQALCAYQAEPNT (SEQ ID NO: 19);
(3) AYQAEPNTCATAQGEGNIK (SEQ ID NO: 20);
(4) WQALCAYQAEPNTCATAQ (SEQ ID NO: 21); or
(5) LAKEWQALCAYQAEPNTCATAQGE (SEQ ID NO: 22).

6. The isolated peptide of claim 5, consisting of the amino acid sequence AYQAEPNTCATAQ (SEQ ID NO: 17).

7. The isolated peptide of claim 5, consisting of the amino acid sequence LAKEWQALCAYQAEPNT (SEQ ID NO: 19).

8. The isolated peptide of claim 5, consisting of the amino acid sequence AYQAEPNTCATAQGEGNIK (SEQ ID NO: 20).

9. The isolated peptide of claim 5, consisting of the amino acid sequence WQALCAYQAEPNTCATAQ (SEQ ID NO: 21).

10. The isolated peptide of claim 5, consisting of the amino acid sequence LAKEWQALCAYQAEPNTCATAQGE (SEQ ID NO: 22).

11. An isolated peptide consisting of the amino acid sequence KLKVESSPSRSDYINAS (SEQ ID NO: 40).

12. A method of diagnosing insulin dependent diabetes mellitus (IDDM) comprising:
(a) providing CD4 lymphocytes from an individual suspected of having or being susceptible to IDDM;
(b) providing a population of APCs which bear on their surface a class II MHC molecule of an allele identical to one expressed by said individual, the population of APCs having been contacted with an IA-2 peptide and the class II MHC molecule being bound to the IA-2 peptide;
(c) contacting the population of APCs of (b) with the CD4 lymphocytes of (a); and
(d) determining whether the CD4 lymphocytes recognize the class II MHC-bound peptide, as an indication that the individual has or is susceptible to IDDM,
Wherein said IA-2 peptide has an amino acid sequence selected from the group consisting of:
YLKNVQTQETRTL (SEQ ID NO: 10); YLKNVQTQETRTLTQ (SEQ ID NO: 13); FYLKNVQTQETRTLTQFHF (SEQ ID NO: 16); AYQAEPNTCATAQ (SEQ ID NO: 17); LAKEWQALCAYQAEPNT (SEQ ID NO: 19); AYQAEPNTCATAQGEGNIK (SEQ ID NO: 20); WQALCAYQAEPNTCATAQ (SEQ ID NO: 21); LAKEWQALCAYQAEPNTCATAQGE (SEQ ID NO: 22); and KLKVESSPSRSDYINAS (SEQ ID NO: 40).

13. The isolated of any one of claims 1 to 11 for use in protecting a subject from insulin dependent diabetes mellitus (IDDM) or the pathogenic symptoms of IDDM.

## Patentansprüche

1. Isoliertes Peptid, das an HLA-DR4 bindet, bestehend aus der Aminosäuresequenz:
(1) YLKNVQTQETRTL (SEQ ID NO: 10);
(2) YLKNVQTQETRTLTQ (SEQ ID NO: 13); oder
(3) FYLKNVQTQETRTLTQFHF (SEQ ID NO: 16).

2. Isoliertes Peptid nach Anspruch 1, bestehend aus der Aminosäuresequenz YLKNVQTQETRTL (SEQ ID NO: 10).

3. Isoliertes Peptid nach Anspruch 1, bestehend aus der Aminosäuresequenz YLKNVQTQETRTLTQ (SEQ ID NO: 13).

4. Isoliertes Peptid nach Anspruch 1, bestehend aus der Aminosäuresequenz FYLKNVQTQETRTLTQFHF (SEQ ID NO: 16).

5. Isoliertes Peptid, das an HLA-DR4 bindet, bestehend aus der Aminosäuresequenz:
(1) AYQAEPNTCATAQ (SEQ ID NO: 17);
(2) LAKEWQALCAYQAEPNT (SEQ ID NO: 19);
(3) AYQAEPNTCATAQGEGNIK (SEQ ID NO: 20);
(4) WQALCAYQAEPNTCATAQ (SEQ ID NO: 21); oder
(5) LAKEWQALCAYQAEPNTCATAQGE (SEQ ID NO: 22).

6. Isoliertes Peptid nach Anspruch 5, bestehend aus der Aminosäuresequenz AYQAEPNTCATAQ (SEQ ID NO: 17).

7. Isoliertes Peptid nach Anspruch 5, bestehend aus der Aminosäuresequenz LAKEWQALCAYQAEPNT (SEQ ID NO: 19).

8. Isoliertes Peptid nach Anspruch 5, bestehend aus der Aminosäuresequenz AYQAEPNTCATAQGEGNIK (SEQ ID NO: 20).

9. Isoliertes Peptid nach Anspruch 5, bestehend aus der Aminosäuresequenz WQALCAYQAEPNTCATAQ (SEQ ID NO: 21).

10. Isoliertes Peptid nach Anspruch 5, bestehend aus der Aminosäuresequenz LAKEWQALCAYQAEPNTCATAQGE (SEQ ID NO: 22).

11. Isoliertes Peptid bestehend aus der Aminosäuresequenz KLKVESSPSRSDYINAS (SEQ ID NO: 40).

12. Verfahren zum Diagnostizieren von insulinabhängigem Diabetes mellitus (IDDM) umfassend:
(a) Bereitstellen von CD4-Lymphozyten aus einem Individuum, das verdächtigt wird, IDDM zu haben oder dafür anfällig zu sein;
(b) Bereitstellen einer Population von APCs, welche auf ihrer Oberfläche ein MHC-Klasse-II-Molekül eines Allels, das identisch zu einem, das von dem Individuum exprimiert wird, ist, tragen, wobei die Population von APCs mit einem IA-2-Peptid in Kontakt gebracht worden ist und das MHC-Klasse-II-Molekül an das IA-2-Peptid gebunden ist;
(c) In-Kontakt-Bringen der Population von APCs aus (b) mit den CD4-Lymphozyten aus (a); und
(d) Bestimmen, ob die CD4-Lymphozyten das MHC-Klasse-II-gebundene Peptid erkennen, als eine Anzeige, dass das Individuum IDDM hat oder dafür anfällig ist,
wobei das IA-2-Peptid eine Aminosäuresequenz aufweist, die ausgewählt ist aus der Gruppe bestehend aus:
YLKNVQTQETRTL (SEQ ID NO: 10); YLKNVQTQETRTLTQ (SEQ ID NO: 13); FYLKNVQTQETRTLTQFHF (SEQ ID NO: 16); AYQAEPNTCATAQ (SEQ ID NO: 17); LAKEWQALCAYQAEPNT (SEQ ID NO: 19); AYQAEPNTCATAQGEGNIK (SEQ ID NO: 20); WQALCAYQAEPNTCATAQ (SEQ ID NO: 21); LAKEWQALCAYQAEPNTCATAQGE (SEQ ID NO: 22); und KLKVESSPSRSDYINAS (SEQ ID NO: 40).

13. Isoliertes Peptid nach einem der Ansprüche 1 bis 11 zur Verwendung zum Schützen eines Subjekts vor insulinabhängigem Diabetes mellitus (IDDM) oder den pathogenen Symptomen von IDDM.

## Revendications

1. Peptide isolé qui se lie au HLA-DR4, constitué de la séquence d'acides aminés :
(1) YLKNVQTQETRTL (SEQ ID NO : 10) ;
(2) YLKNVQTQETRTLTQ (SEQ ID NO : 13) ; ou
(3) FYLKNVQTQETRTLTQFHF (SEQ ID NO : 16).

2. Peptide isolé selon la revendication 1, constitué de la séquence d'acides aminés YLKNVQTQETRTL (SEQ ID NO : 10).

3. Peptide isolé selon la revendication 1, constitué de la séquence d'acides aminés YLKNVQTQETRTLTQ (SEQ ID NO : 13).

4. Peptide isolé selon la revendication 1, constitué de la séquence d'acides aminés FYLKNVQTQETRTLTQFHF (SEQ ID NO : 16).

5. Peptide isolé qui se lie au HLA-DR4, constitué de la séquence d'acides aminés :
(1) AYQAEPNTCATAQ (SEQ ID NO : 17) ;
(2) LAKEWQALCAYQAEPNT (SEQ ID NO : 19) ;
(3) AYQAEPNTCATAQGEGNIK (SEQ ID NO : 20) ;
(4) WQALCAYQAEPNTCATAQ (SEQ ID NO : 21) ; ou
(5) LAKEWQALCAYQAEPNTCATAQGE (SEQ ID NO : 22).

6. Peptide isolé selon la revendication 5, constitué de la séquence d'acides aminés AYQAEPNTCATAQ (SEQ ID NO : 17).

7. Peptide isolé selon la revendication 5, constitué de la séquence d'acides aminés LAKEWQALCAYQAEPNT (SEQ ID NO : 19).

8. Peptide isolé selon la revendication 5, constitué de la séquence d'acides aminés AYQAEPNTCATAQGEGNIK (SEQ ID NO : 20).

9. Peptide isolé selon la revendication 5, constitué de la séquence d'acides aminés WQALCAYQAEPNTCATAQ (SEQ ID NO : 21).

10. Peptide isolé selon la revendication 5, constitué de la séquence d'acides aminés LAKEWQALCAYQAEPNTCATAQGE (SEQ ID NO : 22).

11. Peptide isolé constitué de la séquence d'acides aminés KLKVESSPSRSDYINAS (SEQ ID NO : 40).

12. Procédé de diagnostic d'un diabète insulinodépendant (DID) comprenant :
(a) la fourniture de lymphocytes CD4 provenant d'un individu suspecté de souffrir ou d'être sensible à un DID ;
(b) la fourniture d'une population de CPA qui portent sur leur surface une molécule du CMH de classe II d'un allèle identique à un allèle exprimé par ledit individu, la population de CPA ayant été mise en contact avec un peptide IA-2 et la molécule du CMH de classe II étant liée au peptide IA-2 ;
(c) la mise en contact de la population de CPA de (b) avec les lymphocytes CD4 de (a) ; et
(d) la détermination si les lymphocytes CD4 reconnaissent le peptide lié au CMH de classe II, comme indication que l'individu souffre ou est sensible à un DID,
où ledit peptide IA-2 a une séquence d'acides aminés choisie dans le groupe constitué de:
YLKNVQTQETRTL (SEQ ID NO : 10) ; YLKNVQTQETRTLTQ (SEQ ID NO : 13) ; FYLKNVQTQETRTLTQFHF (SEQ ID NO : 16) ; AYQAEPNTCATAQ (SEQ ID NO : 17) ; LAKEWQALCAYQAEPNT (SEQ ID NO : 19) ; AYQAEPNTCATAQGEGNIK (SEQ ID NO : 20) ; WQALCAYQAEPNTCATAQ (SEQ ID NO : 21) ; LAKEWQALCAYQAEPNTCATAQGE (SEQ ID NO : 22) ; et KLKVESSPSRSDYINAS (SEQ ID NO : 40).

13. Peptide isolé selon l'une quelconque des revendications 1 à 11 pour une utilisation dans la protection d'un sujet contre un diabète insulinodépendant (DID) ou les symptômes pathogènes du DID.
